# EUROPEAN PATENT SPECIFICATION

(11) **EP 4 077 617 B1**
(45) Date of publication and mention of the grant of the patent: **18.03.2026**
(21) Application number: 20841858.2
(22) Date of filing: 18.12.2020
(51) Int. Cl.: C11D 3/20, C11D 3/38, C11D 3/386

(54) **STABILIZED LIQUID BORON-FREE ENZYME COMPOSITIONS**
STABILISIERTE FLÜSSIGE BORFREIE ENZYMZUSAMMENSETZUNGEN
COMPOSITIONS D'ENZYMES LIQUIDES STABILISÉES SANS BORE

(30) Priority: 20.12.2019 WO PCT/CN2019/126886
(43) Date of publication of application: 26.10.2022
(73) Proprietor: Novozymes A/S, 2880 Bagsværd (DK)
(72) Inventor: PEDERSEN, Asbjørn, Toftgaard, 2880 Bagsvaerd (DK); LIU, Zoe, Beijing 100085 (CN)
(74) Representative: NVS EPO Representatives
(86) International application number: PCT/CN2020/137638
(87) International publication number: WO 2021/121394

(56) References cited:
- WO-A1-2017/192417
- WO-A1-97/00992
- WO-A1-97/27354
- DE-A1- 102017 220 084

## Description

### FIELD OF THE INVENTION

The present invention relates to liquid boron-free concentrated enzyme compositions comprising 1,2-pentanediol, 1,2-hexanediol, 1,2-heptanediol, or 1,2-octanediol, which are physically and microbially stable.

### BACKGROUND

Industrial enzymes are used in many different industries, such as household care, food, feed, and biofuels, and are supplied as both solid and liquid products. When liquid enzyme products are shipped across the world, and/or stored in warehouses, it is important that the products are sufficiently stable to maintain specifications, even when they reach the customers a long time after production. Stability includes both enzyme stability, physical stability, and microbial stability.

Microbial stability of liquid enzyme products is traditionally achieved by using preservation agents. Many different preservation agents are known, but since they act by exerting a biocidal effect, there is a desire not to use preservation agents, if possible; in particular in the food industry.

However, the choice of formulation ingredients used to develop such preservative-free and microbially stable formulations is not a simple one, because it will also affect both the enzyme stability and physical stability of the final liquid product, due to (in)compatibility issues.

WO 97/27354 discloses a vapor-releasing containment bag and compositions for fabric cleaning/refreshment in a hot air clothes dryer.

WO 97/00992 discloses a dry cleaning composition and process comprising lipase, protease and/or amylase enzymes, which are applied to soiled fabrics in the presence of an organic solvent, such as butoxy propoxy propanol.

WO 2017/192417 discloses enzymatic cleaning compositions including an enzyme, surfactant, cationic antimicrobial and antimicrobial lipid, which are particularly useful for medical instrument cleaning.

### SUMMARY OF THE INVENTION

The present invention provides, in a first aspect, a liquid boron-free enzyme composition, comprising
(a) 0.5-25% w/w of active enzyme protein,
(b) 0.05-10% w/w of aliphatic 1,2-diols selected from the group consisting of 1,2-pentanediol, 1,2-hexanediol, 1,2-heptanediol, and 1,2-octanediol,
(c) less than 15% w/w of one or more inorganic salts selected from the group consisting of Na, K, NH₄, Ca, Mg, and Zn salts of mono- or divalent anions, and
(d) 50-98% w/w water;

wherein the composition is free of perfume, and
wherein the turbidity of the composition is less than 20 NTU.

Other aspects and embodiments of the invention are apparent from the description and examples.

Unless otherwise indicated, or if it is apparent from the context that something else is meant, all percentages are percentage by weight (% w/w).

### DETAILED DESCRIPTION

We have found that aliphatic alcohols, specifically 1,2-diols, more specifically 1,2-pentanediol, 1,2-hexanediol, 1,2-heptanediol and 1,2-octanediol, act as multifunctional additives in liquid enzyme formulations. These alcohols improve enzyme stability, physical stability, solubility of enzyme protein, and microbial stability.

Thus, we have found that it is possible to prepare preservative-free liquid enzyme products that maintains microbial stability, while not weakening the enzyme stability or the physical stability, by using 1,2-pentanediol, 1,2-hexanediol, 1,2-heptanediol, and/or 1,2-octanediol. The liquid composition maintains enzymatic stability while being substantially free of commonly used boron-based enzyme stabilizers, as shown in for example WO 2018/099762. Boron based stabilizers are generally undesirable and, for example, boric acid has been classified as reprotoxic in the EU REACH classification.

By "boron-free" is meant that no boron-containing compounds are purposefully added to the formulation, but yet it is understood that trace amounts of boron compounds may be present as impurities or as process/stability agents in other additives, i.e. the composition contain less than 500 ppm by weight of the composition of boron compounds. Examples of boron containing compounds include boric acid, boric oxide, borax, alkali metal borates (such as sodium ortho-, meta- and pyroborate and sodium pentaborate), boronic acids, phenylboronic acids (like 4-formylphenylboronic acid), and derivatives and mixtures thereof.

Microbial stability is the ability to resist microbial growth. This may be evaluated by inoculating the liquid composition with microorganisms and measure the subsequent growth of the microorganisms to confirm that they are not proliferating, or growth is reduced or inhibited.

Physical stability is the ability to maintain a transparent, preferably clear, composition. This is measured as turbidity or haziness, by using a nephelometer to measure NTU to determine light scattering.

Enzymatic stability is the ability to maintain enzymatic activity after storage. This may be determined by measuring the enzymatic activity before and after storage (for example, 4 weeks storage at 25°C) to determine how much activity is lost. For practical purposes, the residual activity may be determined by comparing the activity of a stored sample and a frozen reference sample, which are analyzed at the same time to eliminate analytical day-to-day variation.

### Liquid enzyme composition

The liquid enzyme compositions of the invention are liquid boron-free enzyme compositions, comprising
(a) 0.5-25% w/w of active enzyme protein,
(b) 0.05-10% w/w of aliphatic 1,2-diols selected from the group consisting of 1,2-pentanediol, 1,2-hexanediol, 1,2-heptanediol, and 1,2-octanediol,
(c) less than 15% w/w of one or more inorganic salts selected from the group consisting of Na, K, NH₄, Ca, Mg, and Zn salts of mono- or divalent anions, and
(d) 50-98% w/w water;

wherein the composition is free of perfume, and
wherein the turbidity of the composition is less than 20 NTU.

The liquid enzyme compositions of the invention are enzyme products used for delivering enzyme into industrial processes, such as food, feed, biofuel or detergent production; or industrial treatment processes; and consist of enzyme and a liquid delivery vehicle. This is reflected by the high concentration of active enzyme protein, which is the primary active ingredient in the compositions. The liquid delivery vehicle mainly consists of water and other hydrophilic solvents, like polyols, but may also include stabilizers and/or other minors.

Thus, the liquid enzyme compositions are free of (comprise 0% w/w) perfume and optical brightener (fluorescent whitening agent); and comprise less than 0.1% w/w of strong sequestering builder, and less than 1% w/w of hydrophobic solvent (not including the aliphatic 1,2-diols as used according to the invention); all of which are commonly used in consumer products, like laundry prespotters, or laundry or dish wash detergents. The enzyme compositions may also contain less than 3% w/w surfactant, preferably less than 2% w/w or less than 1% w/w surfactant. At least half, or essentially all, of the surfactant may be nonionic surfactant.

Strong sequestering builders are widely used in liquid detergent products for laundry/dishwash and are described on page 13-14 of WO 2014/173980. They are high efficiency chelators that can bind divalent cations (in particular Ca²⁺) strongly without forming any significant amount of precipitate. Examples of strong sequestering builders include EDTA, EDTMP, NTMP, DTPMP, MGDA, NTA, HEDP, STPP, IDS, GLDA, and salts thereof.

Hydrophobic solvents are often used in laundry prespotters to dissolve stains of a hydrophobic nature. Hydrophobic solvents are defined herein by the partition coefficient, where logP_{1-octanol/water} is higher than 0.5 at 25°C.

The liquid enzyme compositions have improved physical stability after storage (such as 4 weeks storage at 25°C), as compared to corresponding liquid compositions without the aliphatic 1,2-diols.

The liquid enzyme compositions exhibit improved microbial stability, as compared to corresponding liquid compositions without the aliphatic 1,2-diols. Microbial stability is evaluated by measuring CFU (colony forming units) per mL, using standard microbiological methods. After inoculating the composition with microorganisms to a total of 10⁵ CFU/mL, the microbial growth may be reduced to less than 10³ CFU/mL, preferably less than 10² CFU/mL, after 4 weeks storage at 25°C.

In an embodiment, the composition is (essentially) free of benzoates, sorbates, sulfites, phenoxyethanol, and isothiozolinones (like methylisothiazolinone, chloromethylisothiazolinone, benzisothiazolinone, octylisothiazolinone, dichlorooctylisothiazolinone, and butyl benzisothiazolinone).

As mentioned above, the liquid composition also maintains excellent enzymatic stability. The residual enzymatic activity may be at least 90% after 4 weeks storage at 25°C.

The liquid enzyme composition comprises more than 50% w/w (such as 50-98% w/w) of water; preferably more than 60% w/w (such as 60-98% w/w) of water, more preferably more than 70% w/w (such as 70-98% w/w) of water, and most preferably more than 80% w/w (such as 80-98% w/w) of water.

### Enzyme

The enzymes used in the compositions of the invention are catalytic proteins, and the term "active enzyme protein" is defined herein as the amount of catalytic protein(s), which exhibits enzymatic activity. This can be determined using an activity based analytical enzyme assay. In such assays, the enzyme typically catalyzes a reaction generating a colored compound. The amount of the colored compound can be measured and correlated to the concentration of the active enzyme protein. This technique is well-known in the art.

The enzyme(s) may be one or more (detergent) enzymes, such as selected from the group consisting of protease, lipase, cutinase, amylase, carbohydrase, cellulase, pectinase, mannanase, arabinase, galactanase, xylanase, nuclease (DNase, RNase), dispersin, catalase, perhydrolase, and oxidase (such as laccase and/or peroxidase). More preferred detergent enzymes are selected from the group consisting of protease, lipase, amylase, cellulase, pectinase, mannanase, xylanase, nuclease (DNase, RNase), dispersin, catalase, and perhydrolase.

The enzyme may be a naturally occurring enzyme of bacterial or fungal origin, or it may be a variant derived from one or more naturally occurring enzymes by gene shuffling and/or by substituting, deleting or inserting one or more amino acids. Chemically modified or protein engineered mutants are included.

The liquid enzyme composition contains at least one enzyme in an amount of 0.5-25% w/w active enzyme protein; and more preferably in an amount of 0.5-20% w/w active enzyme protein.

### Proteases

Suitable proteases may be of any origin, but are preferably of bacterial or fungal origin, optionally in the form of protein engineered or chemically modified mutants. The protease may be an alkaline protease, such as a serine protease or a metalloprotease. A serine protease may for example be of the S1 family, such as trypsin, or the S8 family such as a subtilisin. A metalloprotease may for example be a thermolysin, e.g. from the M4 family, or another metalloprotease such as those from the M5, M7 or M8 families.

The term "subtilases" refers to a sub-group of serine proteases according to Siezen et al., Protein Eng. 4 (1991) 719-737 and Siezen et al., Protein Sci. 6 (1997) 501-523. Serine proteases are a subgroup of proteases characterized by having a serine in the active site, which forms a covalent adduct with the substrate. The subtilases may be divided into six subdivisions, the Subtilisin family, the Thermitase family, the Proteinase K family, the Lantibiotic peptidase family, the Kexin family and the Pyrolysin family.

Although proteases suitable for detergent use may be obtained from a variety of organisms, including fungi such as *Aspergillus*, detergent proteases have generally been obtained from bacteria and in particular from *Bacillus.* Examples of *Bacillus* species from which subtilases have been derived include *Bacillus lentus, Bacillus alkalophilus, Bacillus subtilis, Bacillus amyloliquefaciens, Bacillus licheniformis, Bacillus pumilus* and *Bacillus gibsonii.* Particular subtilisins include *subtilisin lentus*, *subtilisin* Novo, *subtilisin* Carlsberg, *subtilisin* BPN', *subtilisin* 309, *subtilisin* 147 and *subtilisin* 168 and e.g. protease PD138 (described in WO 93/18140). Other useful proteases are e.g. those described in WO 01/16285 and WO 02/16547.

Examples of trypsin-like proteases include the *Fusarium* protease described in WO 94/25583 and WO 2005/040372, and the chymotrypsin proteases derived from *Cellumonas* described in WO 2005/052161 and WO 2005/052146.

Examples of metalloproteases include the neutral metalloproteases described in WO 2007/044993 such as those derived from *Bacillus amyloliquefaciens*, as well as e.g. the metalloproteases described in WO 2015/158723 and WO 2016/075078.

Examples of useful proteases are the protease variants described in WO 89/06279 WO 92/19729, WO 96/34946, WO 98/20115, WO 98/20116, WO 99/11768, WO 01/44452, WO 03/006602, WO 2004/003186, WO 2004/041979, WO 2007/006305, WO 2011/036263, WO 2014/207227, WO 2016/087617 and WO 2016/174234. Preferred protease variants may, for example, comprise one or more of the mutations selected from the group consisting of: S3T, V4I, S9R, S9E, A15T, S24G, S24R, K27R, N42R, S55P, G59E, G59D, N60D, N60E, V66A, N74D, S85R, A96S, S97G, S97D, S97A, S97SD, S99E, S99D, S99G, S99M, S99N, S99R, S99H, S101A, V102I, V102Y, V102N, S104A, G116V, G116R, H118D, H118N, A120S, S126L, P127Q, S128A, S154D, A156E, G157D, G157P, S158E, Y161A, R164S, Q176E, N179E, S182E, Q185N, A188P, G189E, V193M, N198D, V199I, Q200L, Y203W, S206G, L211Q, L211D, N212D, N212S, M216S, A226V, K229L, Q230H, Q239R, N246K, S253D, N255W, N255D, N255E, L256E, L256D T268A and R269H, wherein position numbers correspond to positions of the *Bacillus lentus* protease shown in SEQ ID NO: 1 of WO 2016/001449. Protease variants having one or more of these mutations are preferably variants of the *Bacillus lentus* protease (Savinase^{®}, also known as subtilisin 309) shown in SEQ ID NO: 1 of WO 2016/001449 or of the *Bacillus amyloliquefaciens* protease (BPN') shown in SEQ ID NO: 2 of WO 2016/001449. Such protease variants preferably have at least 80% sequence identity to SEQ ID NO: 1 or to SEQ ID NO: 2 of WO 2016/001449.

Another protease of interest is the alkaline protease from *Bacillus lentus* DSM 5483, as described for example in WO 91/02792, and variants thereof which are described for example in WO 92/21760, WO 95/23221, EP 1921147, EP 1921148 and WO 2016/096711.

The protease may alternatively be a variant of the TY145 protease having SEQ ID NO: 1 of WO 2004/067737, for example a variant comprising a substitution at one or more positions corresponding to positions 27, 109, 111, 171, 173, 174, 175, 180, 182, 184, 198, 199 and 297 of SEQ ID NO: 1 of WO 2004/067737, wherein said protease variant has a sequence identity of at least 75% but less than 100% to SEQ ID NO: 1 of WO 2004/067737. TY145 variants of interest are described in e.g. WO 2015/014790, WO 2015/014803, WO 2015/014804, WO 2016/097350, WO 2016/097352, WO 2016/097357 and WO 2016/097354.

Examples of preferred proteases include:
(a) variants of SEQ ID NO: 1 of WO 2016/001449 comprising two or more substitutions selected from the group consisting of S9E, N43R, N76D, Q206L, Y209W, S259D and L262E, for example a variant with the substitutions S9E, N43R, N76D, V205I, Q206L, Y209W, S259D, N261W and L262E, or with the substitutions S9E, N43R, N76D, N185E, S188E, Q191N, A194P, Q206L, Y209W, S259D and L262E, wherein position numbers are based on the numbering of SEQ ID NO: 2 of WO 2016/001449;
(b) a variant of the polypeptide of SEQ ID NO: 1 of WO 2016/001449 with the mutation S99SE, wherein position numbers are based on the numbering of SEQ ID NO: 2 of WO 2016/001449;
(c) a variant of the polypeptide of SEQ ID NO: 1 of WO 2016/001449 with the mutation S99AD, wherein position numbers are based on the numbering of SEQ ID NO: 2 of WO 2016/001449;
(d) a variant of the polypeptide of SEQ ID NO: 1 of WO 2016/001449 with the substitutions Y167A+R170S+A194P, wherein position numbers are based on the numbering of SEQ ID NO: 2 of WO 2016/001449;
(e) a variant of the polypeptide of SEQ ID NO: 1 of WO 2016/001449 with the substitutions S9R+A15T+V68A+N218D+Q245R, wherein position numbers are based on the numbering of SEQ ID NO: 2 of WO 2016/001449;
(f) a variant of the polypeptide of SEQ ID NO: 1 of WO 2016/001449 with the substitutions S9R+A15T+G61E+V68A+A194P+V205I+Q245R+N261D, wherein position numbers are based on the numbering of SEQ ID NO: 2 of WO 2016/001449;
(g) a variant of the polypeptide of SEQ ID NO: 1 of WO 2016/001449 with the substitutions S99D+S101R/E+S103A+V104I+G160S; for example a variant of SEQ ID NO: 1 of WO 2016/001449 with the substitutions S3T+V4I+S99D+S101E+S103A+V104I+G160S+V205I, wherein position numbers are based on the numbering of SEQ ID NO: 2 of WO 2016/001449;
(h) a variant of the polypeptide of SEQ ID NO: 2 of WO 2016/001449 with the substitutions S24G+S53G+S78N+S101N+G128A/S+Y217Q, wherein position numbers are based on the numbering of SEQ ID NO: 2 of WO 2016/001449;
(i) the polypeptide disclosed in GENESEQP under accession number BER84782, corresponding to SEQ ID NO: 302 in WO 2017/210295;
(j) a variant of the polypeptide of SEQ ID NO: 1 of WO 2016/001449 with the substitutions S99D+S101E+S103A+V104I+S156D+G160S+L262E, wherein position numbers are based on the numbering of SEQ ID NO: 2 of WO 2016/001449;
(k) a variant of the polypeptide of SEQ ID NO: 1 of WO 2016/001449 with the substitutions S9R+A15T+G61E+V68A+N76D+S99G+N218D+Q245R, wherein position numbers are based on the numbering of SEQ ID NO: 2 of WO 2016/001449;
(l) a variant of the polypeptide of SEQ ID NO: 1 of WO 2016/001449 with the substitutions V68A+S106A, wherein position numbers are based on the numbering of SEQ ID NO: 2 of WO 2016/001449; and
(m) a variant of the polypeptide of SEQ ID NO: 1 of WO 2004/067737 with the substitutions S27K+N109K+S111E+S171E+S173P+G174K+S175P+F180Y+G182A+L184F+ Q198E+N199+T297P, wherein position numbers are based on the numbering of SEQ ID NO: 1 of WO 2004/067737.

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase^{™}, Polarzyme^{®}, Kannase^{®}, Liquanase^{®}, Liquanase^{®} Ultra, Ovozyme^{®}, Coronase^{®}, Coronase^{®} Ultra, Blaze^{®}, Blaze Evity^{®} 100T, Blaze Evity^{®} 125T, Blaze Evity^{®} 150T, Blaze Evity^{®} 200T, Neutrase^{®}, Everlase^{®}, Esperase^{®}, Progress^{®} Uno, Progress^{®} In and Progress^{®} Excel (Novozymes A/S), those sold under the tradename Maxatase^{™}, Maxacal^{™}, Maxapem^{®}, Purafect^{®} Ox, Purafect^{®} OxP, Puramax^{®}, FN2^{™}, FN3^{™}, FN4^{ex™}, Excellase^{®}, Excellenz^{™} P1000, Excellenz^{™} P1250, Eraser^{™}, Preferenz^{®} P100, Purafect Prime, Preferenz P110^{™}, Effectenz P1000^{™}, Purafect^{®}, Effectenz P1050^{™}, Purafect^{®} Ox, Effectenz^{™} P2000, Purafast^{™}, Properase^{®}, Opticlean^{™} and Optimase^{®} (Danisco/DuPont), BLAP (sequence shown in Figure 29 of US 5352604) and variants hereof (Henkel AG), and KAP (*Bacillus alkalophilus* subtilisin) from Kao.

### Lipases and Cutinases

Suitable lipases and cutinases include those of bacterial or fungal origin. Chemically modified or protein engineered mutant enzymes are included. Examples include lipase from *Thermomyces*, e.g. from *T. lanuginosus* (previously named *Humicola lanuginosa*) as described in EP258068 and EP305216, cutinase from *Humicola*, e.g. *H. insolens* (WO96/13580), lipase from strains of *Pseudomonas* (some of these now renamed to *Burkholderia*), e.g. *P. alcaligenes* or *P*. *pseudoalcaligenes* (EP218272), *P. cepacia* (EP331376), *P*. *sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase^{™}, Lipex^{™}, Lipolex^{™} and Lipoclean^{™} (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M*. *smegmatis* perhydrolase in particular the S54V variant used in the commercial product Gentle Power Bleach from Huntsman Textile Effects Pte Ltd (WO10/100028).

### Amylases

Suitable amylases may be an alpha-amylase or a glucoamylase and may be of bacterial or fungal origin. Chemically modified or protein engineered mutants are included. Amylases include, for example, alpha-amylases obtained from *Bacillus*, *e.g*., a special strain of *Bacillus licheniformis*, described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase derived from *B*. *amyloliquefaciens* shown in SEQ ID NO: 6 of WO 2006/066594 and residues 36-483 of the *B*. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase derived from *B*. *amyloliquefaciens* shown in SEQ ID NO: 6 of WO 2006/066594 and residues 36-483 of SEQ ID NO: 4 are those having the substitutions:
M 197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:
E187P+I203Y+G476K
E187P+I203Y+R458N+T459S+D460T+G476K
wherein the variants optionally further comprises a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, I181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of I181 and/or G182. Most preferred amylase variants of SEQ ID NO: 1 are those having the substitutions:
N21D+D97N+V128I
wherein the variants optionally further comprise a substitution at position 200 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl^{™}, Termamyl^{™}, Fungamyl^{™}, Stainzyme ^{™}, Stainzyme Plus^{™}, Natalase^{™}, Liquozyme X and BAN^{™} (from Novozymes A/S), and Rapidase^{™}, Purastar^{™}/Effectenz^{™}, Powerase, Preferenz S1000, Preferenz S100 and Preferenz S110 (from Genencor International Inc./DuPont).

### Cellulases

Suitable cellulases include mono-component and mixtures of enzymes of bacterial or fungal origin. Chemically modified or protein engineered mutants are also contemplated. The cellulase may for example be a mono-component or a mixture of mono-component endo-1,4-beta-glucanase also referred to as endoglucanase.

Suitable cellulases include those from the genera *Bacillus*, *Pseudomonas*, *Humicola*, *Myceliophthora, Fusarium*, *Thielavia*, *Trichoderma*, and *Acremonium.* Exemplary cellulases include a fungal cellulase from *Humicola insolens* (US 4,435,307) or from *Trichoderma*, e.g. *T. reesei* or *T. viride.* Other suitable cellulases are from *Thielavia e.g. Thielavia terrestris* as described in WO 96/29397 or the fungal cellulases produced from *Myceliophthora thermophila* and *Fusarium oxysporum* disclosed in US 5,648,263, US 5,691,178, US 5,776,757, WO 89/09259 and WO 91/17244. Also relevant are cellulases from *Bacillus* as described in WO 02/099091 and JP 2000210081. Suitable cellulases are alkaline or neutral cellulases having care benefits. Examples of cellulases are described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307.

Other cellulases are endo-beta-1,4-glucanase enzyme having a sequence of at least 97% identity to the amino acid sequence of position 1 to position 773 of SEQ ID NO:2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Carezyme^{®}, Carezyme^{®} Premium, Celluzyme^{®}, Celluclean^{®}, Celluclast^{®}, Endolase^{®}, Renozyme^{®}; Whitezyme^{®} Celluclean^{®} Classic, Cellusoft^{®} (Novozymes A/S), Puradax^{®}, Puradax HA, and Puradax EG (available from Genencor International Inc.) and KAC-500(B)^{™} (Kao Corporation).

### Mannanases

Suitable mannanases include those of bacterial or fungal origin. Chemically or genetically modified mutants are included. The mannanase may be an alkaline mannanase of Family 5 or 26. It may be a wild-type from *Bacillus* or *Humicola*, particularly *B*. *agaradhaerens, B. licheniformis, B. halodurans, B. clausii*, or *H. insolens.* Suitable mannanases are described in WO 1999/064619. A commercially available mannanase is Mannaway (Novozymes A/S).

### Nucleases

Suitable nucleases include deoxyribonucleases (DNases) and ribonucleases (RNases) which are any enzyme that catalyzes the hydrolytic cleavage of phosphodiester linkages in the DNA or RNA backbone respectively, thus degrading DNA and RNA. There are two primary classifications based on the locus of activity. Exonucleases digest nucleic acids from the ends. Endonucleases act on regions in the middle of target molecules. The nuclease is preferably a DNase, which is preferable is obtainable from a microorganism, preferably a bacterium; in particular a DNase which is obtainable from a species of *Bacillus* is preferred; in particular a DNase which is obtainable from *Bacillus cibi*, *Bacillus subtilis* or *Bacillus licheniformis* is preferred. Examples of such DNases are described in WO 2011/098579, WO2014/087011 and WO2017/060475.

### Dispersins

Suitable dispersins are polypeptides having hexosaminidase activity, EC 3.2.1.- that catalyzes the hydrolysis of β-1,6-glycosidic linkages of N-acetyl-glucosamine polymers (poly-N-acetylglucosamine) found, *e.g*., in biofilm.

### Peroxidases/Oxidases

A suitable peroxidase is preferably a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis*, *e.g*., from *C*. *cinerea* (EP 179,486), and variants thereof as those described in WO 93/24618, WO 95/10602, and WO 98/15257.

Suitable peroxidases also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions. The haloperoxidase may be a chloroperoxidase. Preferably, the haloperoxidase is a vanadium haloperoxidase, *i.e*., a vanadate-containing haloperoxidase. In a preferred method the vanadate-containing haloperoxidase is combined with a source of chloride ion.

Suitable oxidases include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

### Protease stabilizers/inhibitors

Proteases, as described above, may be stabilized using compounds that act by temporarily reducing the proteolytic activity (reversible inhibitors).

Thus, the composition of the invention may also include a protease inhibitor/stabilizer, which is a reversible inhibitor of protease activity, *e.g*., serine protease activity. Preferably, the protease inhibitor is a (reversible) subtilisin protease inhibitor. In particular, the protease inhibitor may be a peptide aldehyde, or a derivative thereof. Examples of protease inhibitors are shown in, for example, WO 2009/118375, WO 2010/055052, and WO 2013/004636.

Antioxidants or reducing agents like sulfite, thiosulfate, nitrite, ascorbic acid/ascorbate etc. are also frequently used to stabilize enzymes (and the water phase in general).

### Aliphatic 1,2-diols

The aliphatic 1,2-diols used according to the invention are selected from the group consisting of 1,2-pentanediol, 1,2-hexanediol, 1,2-heptanediol, and 1,2-octanediol. In particular, the aliphatic 1,2-diols are 1,2-hexanediol and/or 1,2-octanediol.

The aliphatic 1,2-diols are used in an amount of more than 0.05% w/w, and less than 10% w/w; preferably 0.05-8% w/w; and most preferably 0.05-5% w/w.

### Polyol

The liquid composition may contain more than 5% w/w (such as 5-30% w/w, 5-40% w/w, or 5-50% w/w) of one or more polyols, preferably more than 10% w/w (such as 10-30% w/w, 10-40% w/w, or 10-50% w/w) of one or more polyols, and most preferably more than 20% w/w (such as 20-50% w/w) of one or more polyols - excluding the aliphatic 1,2-diols mentioned above.

Polyols (or polyhydric alcohols) according to the invention are alcohols with two or more hydroxyl groups. The polyols typically have a molecular weight lower than 500 g/mol.

Polyols include suitable sugar polyols, such as mono- and disaccharides, like glucose, fructose, galactose, sucrose, lactose, maltose, and trehalose.

Polyols also include suitable non-sugars polyols, such as glycerol, ethylene glycol, diethylene glycol, triethylene glycol, propylene glycol, dipropylene glycol, tripropylene glycol, polyethylene glycol (PEG), and sugar alcohols. The polyethylene glycol may have an average molecular weight at or below about 500. Examples of sugar alcohols are sorbitol, mannitol, erythritol, dulcitol, inositol, xylitol and adonitol.

### Inorganic salt

Salts are commonly used in liquid enzyme formulations; however, we have observed that when more than 15% w/w salt is used in the liquid formulation of the invention, it is detrimental to the physical stability. Thus, the liquid enzyme composition comprises less than 15% w/w of one or more inorganic salts, preferably less than 10% w/w of one or more inorganic salts, and more preferably less than 5% w/w of one or more inorganic salts.

The inorganic salts are selected from the group consisting of Na, K, NH₄, Ca, Mg, and Zn salts of mono- or divalent anions. Examples of anions include chloride, sulphate, nitrate, phosphate, formate, and acetate.

### Detergent compositions

In one embodiment, the invention is directed to the preparation of liquid detergent compositions made by adding the liquid enzyme composition of the invention to a detergent premix comprising one or more additional cleaning or detergent composition components. The choice of additional components is within the skill of the artisan and includes conventional ingredients, including the exemplary non-limiting components set forth below.

The choice of additional detergent components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

In one embodiment, the invention is directed to an ADW (Automatic Dish Wash) compositions comprising an enzyme of the present invention in combination with one or more additional ADW composition components. The choice of additional components is within the skill of the artisan and includes conventional ingredients, including the exemplary non-limiting components set forth below.

### Surfactants

The cleaning composition may comprise one or more surfactants, which may be anionic and/or cationic and/or non-ionic and/or semi-polar and/or zwitterionic, or a mixture thereof. In a particular embodiment, the detergent composition includes a surfactant system (comprising more than one surfactant) e.g. a mixture of one or more nonionic surfactants and one or more anionic surfactants. In one embodiment the detergent comprises at least one anionic surfactant than at least one non-ionic surfactant, the weight ratio of anionic to nonionic surfactant may be from 10:1 to 1:10. In one embodiment the amount of anionic surfactant is higher than the amount of non-ionic surfactant e.g. the weight ratio of anionic to non-ionic surfactant may be from 10:1 to 1.1:1 or from 5:1 to 1.5:1. The amount of anionic to non-ionic surfactant may also be equal and the weight ratios 1:1. In one embodiment the amount of non-ionic surfactant is higher than the amount of anionic surfactant and the weight ratio may be 1:10 to 1:1.1. Preferably the weight ratio of anionic to non-ionic surfactant is from 10:1 to 1:10, such as from 5:1 to 1:5, or from 5:1 to 1:1.2. Preferably, the weight fraction of non-ionic surfactant to anionic surfactant is from 0 to 0.5 or 0 to 0.2 thus non-ionic surfactant can be present or absent if the weight fraction is 0, but if non-ionic surfactant is present, then the weight fraction of the nonionic surfactant is preferably at most 50% or at most 20% of the total weight of anionic surfactant and non-ionic surfactant. Light duty detergent usually comprises more nonionic than anionic surfactant and there the fraction of non-ionic surfactant to anionic surfactant is preferably from 0.5 to 0.9. The total weight of surfactant(s) is typically present at a level of from about 0.1% to about 60% by weight, such as about 1% to about 40%, or about 3% to about 20%, or about 3% to about 10%. The surfactant(s) is chosen based on the desired cleaning application, and may include any conventional surfactant(s) known in the art. When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, typically available as sodium or potassium salts or salts of monoethanolamine (MEA, 2-aminoethan-1-ol) or triethanolamine (TEA, 2,2',2"-nitrilotriethan-1-ol); in particular, linear alkylbenzenesulfonates (LAS), isomers of LAS such as branched alkylbenzenesulfonates (BABS) and phenylalkanesulfonates; olefin sulfonates, in particular alpha-olefinsulfonates (AOS); alkyl sulfates (AS), in particular fatty alcohol sulfates (FAS), *i.e*., primary alcohol sulfates (PAS) such as dodecyl sulfate; alcohol ethersulfates (AES or AEOS or FES, also known as alcohol ethoxysulfates or fatty alcohol ether sulfates); paraffin sulfonates (PS) including alkane-1-sulfonates and secondary alkanesulfonates (SAS); ester sulfonates, including sulfonated fatty acid glycerol esters and alpha-sulfo fatty acid methyl esters (alpha-SFMe or SES or MES); alkyl- or alkenylsuccinic acids such as dodecenyl/tetradecenyl succinic acid (DTSA); diesters and monoesters of sulfosuccinic acid; fatty acid derivatives of amino acids. Furthermore, salts of fatty acids (soaps) may be included.

When included therein the detergent will usually contain from about 1% to about 40% by weight of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO) e.g. the AEO-series such as AEO-7, alcohol propoxylates, in particular propoxylated fatty alcohols (PFA), ethoxylated and propoxylated alcohols, alkoxylated fatty acid alkyl esters, such as ethoxylated and/or propoxylated fatty acid alkyl esters (in particular methyl ester ethoxylates, MEE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.

When included therein the detergent will usually contain from about 0.01 to about 10 % by weight of a semipolar surfactant. Non-limiting examples of semipolar surfactants include amine oxides (AO) such as alkyldimethylamine oxides, in particular N-(coco alkyl)-N,N-dimethylamine oxide and N-(tallow-alkyl)-N,N-bis(2-hydroxyethyl)amine oxide, and combinations thereof.

When included therein the detergent will usually contain from about 0.01 % to about 10 % by weight of a zwitterionic surfactant. Non-limiting examples of zwitterionic surfactants include betaines such as alkyldimethylbetaines, sulfobetaines, and combinations thereof.

Additional bio-based surfactants may be used e.g. wherein the surfactant is a sugar-based non-ionic surfactant which may be a hexyl-β-D-maltopyranoside, thiomaltopyranoside or a cyclic-maltopyranoside, such as described in EP2516606 B1.

### Builders and Co-Builders

The detergent composition may contain about 0-65% by weight, such as about 5% to about 50% of a detergent builder or co-builder, or a mixture thereof. In a dish wash detergent, the level of builder is typically in the range 40-65%, particularly in the range 50-65%. The builder and/or co-builder may particularly be a chelating agent that forms water-soluble complexes with Ca and Mg. Any builder and/or co-builder known in the art for use in cleaning detergents may be utilized.

Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Clariant), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain from about 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diylbis(phosphonic acid (HEDP), ethylenediaminetetramethylenetetrakis(phosphonic acid) (EDTMPA), diethylenetriaminepentamethylenepentakis(phosphonic acid) (DTMPA or DTPMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)aspartic acid (SMAS), N-(2-sulfoethyl)aspartic acid (SEAS), N-(2-sulfomethyl)glutamic acid (SMGL), N-(2-sulfoethyl)glutamic acid (SEGL), N-methyliminodiacetic acid (MIDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA) , taurine-N,N-diacetic acid (TUDA) and sulfomethyl-N,N-diacetic acid (SMDA), N-(2-hydroxyethyl)ethylenediamine-N,N',N"-triacetic acid (HEDTA), diethanolglycine (DEG), aminotrimethylenetris(phosphonic acid) (ATMP), and combinations and salts thereof. Further exemplary builders and/or co-builders are described in, e.g., WO 09/102854, US 5977053.

### Bleaching Systems

The cleaning composition may contain 0-50% by weight, such as 1-40%, such as 1-30%, such as about 1% to about 20%, of a bleaching system. Any oxygen-based bleaching system comprising components known in the art for use in cleaning detergents may be utilized. Suitable bleaching system components include sources of hydrogen peroxide; peracids and sources of peracids (bleach activators); and bleach catalysts or boosters.

Suitable sources of hydrogen peroxide are inorganic persalts, including alkali metal salts such as sodium percarbonate and sodium perborates (usually mono- or tetrahydrate), and hydrogen peroxide-urea.

Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone^{®}) or alkaline earth-metal salts.

Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

### Bleach catalysts and boosters

The bleaching system may also include a bleach catalyst or booster. Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal compounds; such as iron or cobalt complexes.

In some embodiments, where a source of a peracid is included, an organic bleach catalyst or bleach booster may be used having one of the following formulae:
(i) 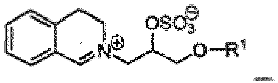
(ii) 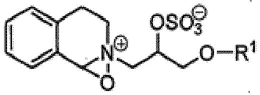
(iii) and mixtures thereof;
wherein R1 is independently a branched alkyl group containing from 9 to 24 carbons or linear alkyl group containing from 11 to 24 carbons, preferably R1 is independently a branched alkyl group containing from 9 to 18 carbons or linear alkyl group containing from 11 to 18 carbons, more preferably R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO 2007/087258, WO 2007/087244, WO 2007/087259, EP 1 867 708 (Vitamin K) and WO 2007/087242.

### Additional enzymes

In addition to the present invention, enzymes may be also added to the detergent also as standard aqueous formulations or slurries, or as granulated products.

### Polymers

The detergent may contain 0.005-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(ethyleneglycol) or poly(ethylene oxide) (PEG or PEO), ethoxylated poly(ethyleneimine), (carboxymethyl)inulin (CMI), carboxylate polymers and polycarboxylates such as polyacrylates, maleic/acrylic acid copolymers, acrylate/styrene copolymers, poly(aspartic) acid, and lauryl methacrylate/acrylic acid copolymers, hydrophobically modified CMC (HM-CMC), silicones, copolymers of terephthalic acid and oligomeric glycols, copolymers of poly(ethylene terephthalate) and poly(oxyethene terephthalate) (PET-POET), poly(vinylpyrrolidone) (PVP), poly(vinylimidazole) (PVI), poly(vinylpyridine-N-oxide) (PVPO or PVPNO) and copoly(vinylimidazole/vinylpyrrolidone) (PVPVI). Suitable examples include PVP-K15, PVP-K30, ChromaBond S-400, ChromaBond S-403E and Chromabond S-100 from Ashland Aqualon, and Sokalan^{®} HP 165, Sokalan^{®} HP 50 (Dispersing agent), Sokalan^{®} HP 53 (Dispersing agent), Sokalan^{®} HP 59 (Dispersing agent), Sokalan^{®} HP 56 (dye transfer inhibitor), Sokalan^{®} HP 66 K (dye transfer inhibitor) from BASF. Further exemplary polymers include sulfonated polycarboxylates, polyethylene oxide and polypropylene oxide (PEO-PPO) and diquaternium ethoxy sulfate. Particularly preferred polymer is ethoxylated homopolymer Sokalan^{®} HP 20 from BASF, which helps to prevent redeposition of soil in the wash liqor. Further exemplary polymers include sulfonated polycarboxylates, ethylene oxide-propylene oxide copolymers (PEO-PPO), copolymers of PEG with and vinyl acetate, and diquaternium ethoxy sulfate or quaternized sulfated ethoxylated hexamethylenediamine. Other exemplary polymers are disclosed in, e.g., WO 2006/130575. Salts of the above-mentioned polymers are also contemplated.

### Adjunct materials

Any detergent components known in the art for use in laundry/ADW/hard surface cleaning detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry/ADW/hard surface cleaning detergents may be utilized. The choice of such ingredients is well within the skill of the artisan.

### Dispersants

The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

### Dye Transfer Inhibiting Agents

The detergent compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of *N-*vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

### Fluorescent whitening agent

The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-d][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

### Soil release polymers

The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Other types of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore, random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

### Anti-redeposition agents

The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

### Rheology Modifiers

The detergent compositions of the present invention may also include one or more rheology modifiers, structurants or thickeners, as distinct from viscosity reducing agents. The rheology modifiers are selected from the group consisting of non-polymeric crystalline, hydroxyfunctional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

### Formulation of detergent products

The detergent composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multi compartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water-soluble acrylate copolymers, methyl cellulose, carboxy methyl cellulose, sodium dextrin, ethyl cellulose, hydroxyethyl cellulose, hydroxypropyl methyl cellulose, maltodextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water-soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water-soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may also be non-aqueous.

### EXAMPLES

Chemicals were commercial products of at least reagent grade.

### EXAMPLE 1

### Improved visual appearance of amylase and protease

The transparency (turbidity) of two commercial liquid protease (Fermax from Novozymes) and amylase (Fortiva Revo from Novozymes) products were evaluated before and after addition of different amounts of 1,2-hexanediol. The turbidity was measured using a nephelometer, as shown in Table 1.

**Table 1. Turbidity after addition of 1,2-hexanediol.**

| Enzyme | 1,2-hexanediol | | |
|---|---|---|---|
| | 0% w/w (reference) | 5% w/w | 10% w/w |
| Amylase | 23.0 NTU | 17.2 NTU | 14.6 NTU |
| Protease | 30.4 NTU | 21.2 NTU (reference) | 11.7 NTU |

The exact nature of the two liquid enzyme products is not important. The data in Table 1 simply shows that even a purified commercial enzyme product, that appears clear, can get an even better transparency (reduced turbidity) by addition of 1,2-hexanediol.

### EXAMPLE 2

### Improved physical stability of lipase and amylase

An aqueous amylase solution was formulated with 40% w/w glycerol at pH 4.8 to a final enzyme protein concentration of 4.3% w/w. A sample was prepared by adding 0.3% w/w 1,2-octanediol to the amylase solution, and the physical stability was evaluated by visual inspection and NTU measurement, as shown in Table 2.

**Table 2. Physical stability of an amylase with and without 1,2-octanediol**

| Storage condition | Evaluation method | 1,2-octanediol | |
|---|---|---|---|
| | | 0% w/w (reference) | 0.3% w/w |
| 4 weeks at 5°C | Visual score | haze | haze |
| | Turbidity | 24.5 NTU | 19.1 NTU |
| 4 weeks at 25°C | Visual score | precipitate | haze |
| | Turbidity | 21.6 NTU | 11.9 NTU |
| 4 weeks at 40°C | Visual score | precipitate | haze |
| | Turbidity | 22.6 NTU | 12.7 NTU |

An aqueous lipase solution was formulated with 15% w/w sorbitol and 6% w/w MPG to a final enzyme protein concentration of 3% w/w. Two samples were prepared by adding 1% w/w 1,2-hexanediol or 0.3% w/w 1,2-octanediol to the lipase solution. The physical stability was evaluated by visual inspection, as shown in Table 3.

**Table 3. Physical stability of a lipase in the presence of 1,2-hexanediol or 1,2-octanediol.**

| Storage condition | Reference | 1,2-hexanediol 1% w/w | 1,2-octanediol 0.3% w/w |
|---|---|---|---|
| 4 weeks at 5°C | clear | clear | clear |
| 4 weeks at 25°C | precipitate | haze | clear |
| 4 weeks at 40°C | precipitate | Precipitate (reference) | haze |

An aqueous protease solution and an aqueous pectate lyase solution were formulated with 10% w/w water, 10% w/w 1,2-hexanediol, or 10% w/w glycerol to a final enzyme protein concentration of 6.8% w/w and 8.1% w/w, respectively. The physical stability was evaluated after storage for 4 weeks at 25°C, by visual inspection and by NTU measurements, as shown in Table 4.

**Table 4. Physical stability of a protease and a pectate lyase in the presence of 1,2-hexanediol or glycerol.**

| Enzyme | Evaluation method | Water 10% w/w (reference) | 1,2-hexanediol 10% w/w | Glycerol 10% w/w (reference) |
|---|---|---|---|---|
| Protease | Visual score | precipitate | clear | precipitate |
| | Turbidity | 143 | 9.4 | 71.2 |
| Pectate lyase | Visual score | precipitate | haze | precipitate |
| | Turbidity | 920 | 31.2 (reference) | 540 |

The data in Tables 2, 3, and 4 show that addition of aliphatic 1,2-diols improved the physical stability of enzyme formulations stored for several weeks at room temperature.

### EXAMPLE 3

### Improved enzyme solubility

An aqueous enzyme solution produced via fermentation of microorganism and recovered by removal of cells were concentrated using ultrafiltration to form a supersaturated protein solution. The solution was cooled below 5°C for more than 24 hours to generate protein precipitate. The protein precipitate was suspended in the cold solution by shaking before use. Water and 1,2-hexanediol or glycerol were added to the protein suspension to obtain a diluted protein suspension with the indicated concentration of 1,2-hexanediol or glycerol while keeping the dilution of the protein suspension constant (70% w/w protein suspension to 30% w/w water/1,2-hexanediol/glycerol). The diluted protein suspensions were mixed for 2 hours at room temperature on a magnetic stirred followed by centrifugation to generate a clear supernatant. The obtained supernatant was analyzed for enzyme activity. The data in Table 5 show how much the enzyme solubility (enzyme activity) has increased compared to the reference sample, which only contains water.

**Table 5. Increase in enzyme solubility at increasing 1,2-hexanediol or glycerol concentration.**

| Enzyme | Water (reference) | 1,2-hexanediol | | Glycerol | |
|---|---|---|---|---|---|
| | | 2% w/w | 5% w/w | 2% w/w | 5% w/w |
| Mannanase | 0% | 7% | 48% | 10% | 41% |
| Protease | 0% | 11% | 47% | 0% | 3% |
| Pectate lyase | 0% | 32% | 66% | 10% | 32% |

The data in Table 5 show that addition of 1,2-hexanediol greatly improves the enzyme solubility.

### EXAMPLE 4

### Improved enzymatic stability of protease

An aqueous protease (subtilisin) solution was produced via fermentation of microorganism, recovered by removal of cells and concentrated using ultrafiltration. Water and 1,2-hexanediol were added to the protease solution to obtain a diluted protease solution with the indicated concentration of 1,2-hexanediol while keeping the dilution of the protease solution constant (70 w/w% protease solution to 30% w/w water/1,2-hexanediol). The diluted protease solutions were incubated in closed vials at -18°C, 25°C and 40°C for 4 weeks. The protease activity was measured in the stored samples and residual activity calculated by using protease activity in samples stored at -18°C as reference.

**Table 6. Residual activity of protease after 4 weeks storage.**

| Storage temperature | 1,2-hexanediol | | |
|---|---|---|---|
| | 0% w/w (reference) | 2% w/w | 5% w/w |
| -18°C | 100% | 100% | 100% |
| 25°C | 32% | 48% | 77% |
| 40°C | 3% | 8% | 14% |

The data in Table 6 show that addition of 1,2-hexanediol improves the enzyme stability. The residual protease activity increased from 32% to 77% after 4 weeks at room temperature upon addition of 1,2-hexanediol.

Thus, the residual enzyme activity increased about 140% by adding 5% w/w of the aliphatic 1,2-diol.

### EXAMPLE 5

### Improved microbial stability of liquid enzyme products

An aqueous protease (subtilisin) solution was formulated with 40% w/w glycerol at pH 6.0 to a final enzyme protein concentration of 5.5% w/w. Two samples were prepared by adding 4% w/w 1,2-hexanediol or 0.3% w/w 1,2-octanediol to the protease solution.

The protease formulations were shown to be microbially robust towards bacteria, lactobacilli as well as yeast and mold. This was done by challenging the formulations with the microorganisms in Table 7. Each of the three bottles in Table 8 were inoculated to a total of 10⁵ CFU/mL of the test microorganisms.

**Table 7. Test microorganisms used in bottles 1-3.**

| **Bottle** | **Test organism** |
|---|---|
| 1 | *Escherichia coli* |
| | *Pseudomonas aeruginosa* |
| | *Salmonella havana* |
| | *Acinetobacter* spp. |
| | *Staphylococcus aureus* |
| | *Staphylococcus xylosus* |
| | *Enterococcus faecium* |
| 2 | *Lactobacillus buchneri* |
| | *Lactobacillus para paracasei* |
| 3 | *Aspergillus niger* |
| | *Candida parapsilosis* |
| | *Candida famata* |

The bottles were analyzed for CFU/mL after 2 and 4 weeks of incubation at 20-25°C. CFU (colony forming units) per mL was measured using standard microbiological methods.

**Table 8. Microbial stability of the liquid enzyme formulations, measured as CFU/mL.**

| **Formulation** | **Bottle** | **0 weeks** | **2 weeks** | **4 weeks** |
|---|---|---|---|---|
| Reference | 1 | 1.0 x 10⁵ | 2.8 x 10⁴ | 7.3 x 10³ |
| | 2 | 1.0 x 10⁵ | 7.0 x 10² | < 1.0 x 10² |
| | 3 | 1.0 x 10⁵ | 4.9 x 10⁴ | 3.7 x 10⁴ |
| 4% 1,2-hexanediol | 1 | 1.0 x 10⁵ | < 1.0 x 10² | < 1.0 x 10² |
| | 2 | 1.0 x 10⁵ | < 1.0 x 10² | < 1.0 x 10² |
| | 3 | 1.0 x 10⁵ | < 1.0 x 10² | < 1.0 x 10² |
| 0.3% 1,2-octanediol | 1 | 1.0 x 10⁵ | 3.0 x 10² | < 1.0 x 10² |
| | 2 | 1.0 x 10⁵ | < 1.0 x 10² | < 1.0 x 10² |
| | 3 | 1.0 x 10⁵ | 7.0 x 10² | < 1.0 x 10² |

The data in Table 8 show that addition of aliphatic 1,2-diols improved the microbial stability/robustness. Contrary to the references, most of the samples with aliphatic 1,2-diols had less than 10² CFU/mL after 2 weeks storage; and after 4 weeks, they were all below 10² CFU/mL.

## Claims

1. A liquid boron-free enzyme composition comprising:
(a) 0.5-25% w/w of active enzyme protein,
(b) 0.05-10% w/w of aliphatic 1,2-diols selected from the group consisting of 1,2-pentanediol, 1,2-hexanediol, 1,2-heptanediol, and 1,2-octanediol, and
(c) less than 15% w/w of one or more inorganic salts selected from the group consisting of Na, K, NH₄, Ca, Mg, and Zn salts of mono- or divalent anions, and
(d) 50-98% w/w of water;
wherein the composition is free of perfume, and
wherein the turbidity of the composition is less than 20 NTU.

2. The composition of claim 1, which further comprises 5-50% w/w of one or more polyols, which are not the aliphatic 1,2-diols.

3. The composition of claim 1, which further comprises 5-40% w/w of one or more polyols, which are not the aliphatic 1,2-diols.

4. The composition of any of claims 1-3, wherein the aliphatic 1,2-diols are 1,2-hexanediol and/or 1,2-octanediol.

5. The composition of any of claims 1-4, which comprises 0.05-5% w/w of the aliphatic 1,2-diols.

6. The composition of any of claims 1-5, which comprises 50-90% w/w of water.

7. The composition of any of claims 1-6, wherein the enzyme is selected from the group consisting of protease, lipase, cutinase, amylase, carbohydrase, cellulase, pectinase, mannanase, arabinase, galactanase, xylanase, nuclease, dispersin, perhydrolase, catalase, and oxidase.

8. The composition of any of claims 1-7, wherein the enzyme is selected from the group consisting of lipase, cutinase, amylase, carbohydrase, cellulase, pectinase, mannanase, arabinase, galactanase, xylanase, nuclease, dispersin, perhydrolase, catalase, and oxidase.

9. The composition of any of claims 1-8, wherein the enzyme is nuclease, preferably DNase.

10. The composition of any of claims 1-9, which is free of optical brightener.

11. The composition of any of claims 1-10, which comprises less than 3% w/w of surfactant.

12. The composition of any of claims 1-11, which comprises less than 0.1% w/w of strong sequestering builders selected from EDTA, EDTMP, NTMP, DTPMP, MGDA, NTA, HEDP, STPP, IDS, GLDA, and/or salts thereof.

13. The composition of any of claims 1-12, which is essentially free of benzoates, sorbates, sulfites, phenoxyethanol, and isothiozolinones.

14. A method for preparing a liquid detergent composition, comprising adding the composition of any of claims 1-13 to a detergent composition premix.

## Patentansprüche

1. Flüssige borfreie Enzymzusammensetzung, umfassend:
(a) 0,5-25% Gew./Gew. aktives Enzymprotein,
(b) 0,05-10% Gew./Gew. aliphatische 1,2-Diole, ausgewählt aus der Gruppe bestehend aus 1,2-Pentandiol, 1,2-Hexandiol, 1,2-Heptandiol und 1,2-Octandiol,
(c) weniger als 15% Gew./Gew. eines oder mehrerer anorganischer Salze, ausgewählt aus der Gruppe bestehend aus Na-, K-, NH₄-, Ca-, Mg- und Zn-Salzen von ein- oder zweiwertigen Anionen, und
(d) 50-98% Gew./Gew. Wasser;
wobei die Zusammensetzung frei von Parfüm ist, und
wobei die Trübung der Zusammensetzung weniger als 20 NTU beträgt.

2. Zusammensetzung nach Anspruch 1, die des Weiteren 5-50% Gew./Gew. eines oder mehrerer Polyole umfasst, die nicht die aliphatischen 1,2-Diole sind.

3. Zusammensetzung nach Anspruch 1, die des Weiteren 5-40% Gew./Gew. eines oder mehrerer Polyole umfasst, die nicht die aliphatischen 1,2-Diole sind.

4. Zusammensetzung nach einem beliebigen der Ansprüche 1-3, wobei die aliphatischen 1,2-Diole 1,2-Hexandiol und/oder 1,2-Octandiol sind.

5. Zusammensetzung nach einem beliebigen der Ansprüche 1-4, die 0,05-5% Gew./Gew. der aliphatischen 1,2-Diole umfasst.

6. Zusammensetzung nach einem beliebigen der Ansprüche 1-5, die 50-90% Gew./Gew. Wasser umfasst.

7. Zusammensetzung nach einem beliebigen der Ansprüche 1-6, wobei das Enzym aus der Gruppe ausgewählt ist, die aus Protease, Lipase, Cutinase, Amylase, Carbohydrase, Cellulase, Pectinase, Mannanase, Arabinase, Galactanase, Xylanase, Nuklease, Dispersin, Perhydrolase, Katalase und Oxidase besteht.

8. Zusammensetzung nach einem beliebigen der Ansprüche 1-7, wobei das Enzym aus der Gruppe ausgewählt ist, die aus Lipase, Cutinase, Amylase, Carbohydrase, Cellulase, Pectinase, Mannanase, Arabinase, Galactanase, Xylanase, Nuklease, Dispersin, Perhydrolase, Katalase und Oxidase besteht.

9. Zusammensetzung nach einem beliebigen der Ansprüche 1-8, wobei das Enzym Nuklease, vorzugsweise DNase, ist.

10. Zusammensetzung nach einem beliebigen der Ansprüche 1-9, die frei von optischen Aufhellern ist.

11. Zusammensetzung nach einem beliebigen der Ansprüche 1-10, die weniger als 3% Gew./Gew. oberflächenaktives Mittel umfasst.

12. Zusammensetzung nach einem beliebigen der Ansprüche 1-11, die weniger als 0,1% Gew./Gew. stark sequesternde Gerüststoffe umfasst, ausgewählt aus EDTA, EDTMP, NTMP, DTPMP, MGDA, NTA, HEDP, STPP, IDS, GLDA und/oder Salzen davon.

13. Zusammensetzung nach einem beliebigen der Ansprüche 1-12, die im Wesentlichen frei von Benzoaten, Sorbaten, Sulfiten, Phenoxyethanol und Isothiazolinonen ist.

14. Verfahren zum Herstellen einer flüssigen Detergenszusammensetzung, umfassend Hinzufügen der Zusammensetzung gemäß einem beliebigen der Ansprüche 1-13 zu einer Detergenszusammensetzungsvormischung.

## Revendications

1. Composition enzymatique liquide exempte de bore, comprenant :
(a) 0,5 à 25 % en p/p de protéine enzymatique active,
(b) 0,05 à 10 % en p/p de 1,2-diols aliphatiques choisis dans le groupe consistant en 1,2-pentanediol, 1,2-hexanediol, 1,2-heptanediol et 1,2-octanediol,
(c) moins de 15 % en p/p d'un ou de plusieurs sels inorganiques choisis dans le groupe consistant en sels de Na, K, NH₄, Ca, Mg et Zn d'anions mono- ou divalents, et
(d) 50 à 98 % en p/p d'eau ;
où la composition est dépourvue de parfum, et
où la turbidité de la composition est inférieure à 20 NTU.

2. Composition selon la revendication 1, qui comprend en outre 5 à 50 % en p/p d'un ou de plusieurs polyols qui ne sont pas les 1,2-diols aliphatiques.

3. Composition selon la revendication 1, qui comprend en outre 5 à 40 % en p/p d'un ou de plusieurs polyols qui ne sont pas les 1,2-diols aliphatiques.

4. Composition selon l'une quelconque des revendications 1 à 3, dans laquelle les 1,2-diols aliphatiques sont le 1,2-hexanediol et/ou le 1,2-octanediol.

5. Composition selon l'une quelconque des revendications 1 à 4, qui comprend 0,05 à 5 % en p/p des 1,2-diols aliphatiques.

6. Composition selon l'une quelconque des revendications 1 à 5, qui comprend 50 à 90 % en p/p d'eau.

7. Composition selon l'une quelconque des revendications 1 à 6, dans laquelle l'enzyme est choisie dans le groupe consistant en protéase, lipase, cutinase, amylase, carbohydrase, cellulase, pectinase, mannanase, arabinase, galactanase, xylanase, nucléase, dispersine, perhydrolase, catalase, et oxydase.

8. Composition selon l'une quelconque des revendications 1 à 7, dans laquelle l'enzyme est choisie dans le groupe consistant en lipase, cutinase, amylase, carbohydrase, cellulase, pectinase, mannanase, arabinase, galactanase, xylanase, nucléase, dispersine, perhydrolase, catalase, et oxydase.

9. Composition selon l'une quelconque des revendications 1 à 8, dans laquelle l'enzyme est la nucléase, de préférence la DNase.

10. Composition selon l'une quelconque des revendications 1 à 9, qui est exempte d'azurant optique.

11. Composition selon l'une quelconque des revendications 1 à 10, qui comprend moins de 3 % en p/p de tensioactif.

12. Composition selon l'une quelconque des revendications 1 à 11, qui comprend moins de 0,1 % en p/p d'agents de détergence à forte séquestration choisis parmi EDTA, EDTMP, NTMP, DTPMP, MGDA, NTA, HEDP, STPP, IDS, GLDA et/ou leurs sels.

13. Composition selon l'une quelconque des revendications 1 à 12, qui est sensiblement exempte de benzoates, sorbates, sulfites, phénoxyéthanol et isothiazolinones.

14. Méthode de préparation d'une composition détergente liquide, comprenant l'addition de la composition selon l'une quelconque des revendications 1 à 13 à un prémélange de composition détergente.
